(19) **Europäisches Patentamt / European Patent Office / Office européen des brevets**

(11) **EP 3 479 862 A1**

(12) # EUROPEAN PATENT APPLICATION
published in accordance with Art. 153(4) EPC

(43) Date of publication:
**08.05.2019 Bulletin 2019/19**

(51) Int Cl.:
**A61M 16/12** *(2006.01)*    **A61M 16/01** *(2006.01)*

(21) Application number: **17810631.6**

(22) Date of filing: **07.06.2017**

(86) International application number:
**PCT/RU2017/000390**

(87) International publication number:
**WO 2017/213556 (14.12.2017 Gazette 2017/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.06.2016 RU 2016123063**

(71) Applicant: **Panin, Aleksandr Andreevich**
**Moscow 123001 (RU)**

(72) Inventor: **Panin, Aleksandr Andreevich**
**Moscow 123001 (RU)**

(54) **METHOD FOR INHALATION EFFECT ON THE BODY, AND APPARATUS FOR IMPLEMENTING SAME**

(57)    The invention relates to medical technology and, specifically, to methods and apparatuses for an inhalation effect on the human body. A method consists in forming a respiratory mixture on the basis of oxygen and/or air, and feeding same to a patient with the addition of anesthetic vapors and/or of an aerosol of medicinal agents. An apparatus comprises a respiratory-mixture formation device with a gas source, a primary unit with a unit for setting the volume of respiratory mixture fed to a patient, which are connected to a primary unit of an inhalation/exhalation line having diverters for connecting to the atmosphere, and which are integrated into a patient tee-fitting, a control module, and means for automatic adjustment and monitoring. The control module, aided by the means for automatic adjustment and monitoring, sets a mode for feeding the respiratory mixture along a rebreathing or non-rebreathing loop, using artificial ventilation or autonomous breathing. The invention allows for increasing the efficiency and broadening the functional capabilities of an inhalation effect.

EP 3 479 862 A1

# Description

## Scope of use

The invention relates to medical technology, specifically to methods and apparatuses for an inhalation effect on the human body during inhalation therapy, intensive treatment, inhalational and non-inhalational anesthesia with respiratory mixtures containing humidified and heated oxygen and/or air while ensuring the possibility of adding the respiratory mixture with liquid anesthetic vapors and/or aerosols of medicinal agents, and may be used for artificial lung ventilation and/or autonomous breathing via a rebreathing or a non-rebreathing loop.

## *Prior art*

A method for formation of various breathing gas mixtures by mixing compressed gases and their subsequent feeding at a controlled flow through the breathing loop to the respiratory tract of a patient via the breathing mask with the possibility to increase the temperature of inhaled mixtures to at least 90 °C and introducing aerosols of medicinal agents into them, wherein the composition of an inhaled mixture and its temperature are monitored with employing a rebreathing loop with removal of carbon dioxide from the inhaled mixture, and also a device for implementing this method are known in the art (see patent RU 2072241, cl. A61M16/00, published on 27.01.1997). However, the known method does not allow for using various gas mixtures during the artificial lung ventilation (ALV), including air and anesthetic vapors into gas mixtures,

and supplying gas mixtures at a flow below 3 1/min for implementing the low-flow rebreathing loop and provides for the connection to the patient only via a facemask. The apparatus for implementing the known method also does not allow for separately creating and stabilizing the flow of the gas mixture and does not provide a high-frequency (HF) ALV and monitoring of ventilation indicators and patient safety in case of failure of power supply and (or) compressed-air supply. There is no possibility for actively mitigating the resistance to autonomous breathing.

A method for controlling the physiological condition of a biological subject using gas mixtures by exposing the biological subject, who is placed in the environment containing at least one gas, to the gas mixture containing oxygen in a cyclic mode with particular parameters and ensuring saturation/desaturation of at least one component of a gas mixture in tissues of the biological subject according to a predetermined algorithm is also known in the art (see patent RU 2291718, cl.A61M 16/12, published on 20.01.2007).

However, owing to the need to place the patient in a particular environment, the known method may not be used in medical treatment facilities in practical cases of inhalation therapy, anesthesia, and intensive treatment; the method is considered for use only in case of autonomous breathing of the patient, and the invention does not describe technical solutions to implement the given method.

In addition, a method for creating an inhalation effect on the human body, consisting in setting the breathing-mixture feeding mode via a control module, forming the respective respiratory mixture with the required concentration of an anesthetic agent, measuring and adjusting the respiratory-mixture flow, and feeding it to a patient and also a device for its implementation are known in the art (see patent US 6553990, cl. A61M 16/10, published on 29.04.2003). However, the known method and device do not allow for varying the modes of feeding a respiratory mixture via a rebreathing or non-rebreathing loop using artificial lung ventilation and/or autonomous breathing.

According to the technical essence, the method closest to the claimed invention is a method for creating an inhalation effect on the human body, consisting in formation of at least a two-component respiratory mixture on the basis of oxygen and/or air, and feeding the mixture to a patient while ensuring the possibility of adding the respiratory mixture with anesthetic vapors and/or an aerosol of medicinal agents (see patent RU 2146536, cl. A61M16/12, published on 20.03.2000). A device for creating an inhalation effect on the human body is known from the same document, consisting of a respiratory-mixture formation device having the possibility to connect at least two gas sources; a primary unit with a unit for setting the volume of respiratory mixture fed to the patient, wherein the primary unit is connected to the primary outlet of the respiratory-mixture formation device via the filling line; inhalation and exhalation lines, which are connected to the primary unit, which have diverters for connecting to the atmosphere, and which are integrated into a patient tee-fitting piece; a control module; and means for automatic adjustment and monitoring.

However, the known method requires the application of cylinders containing precomposed compressed gas mixtures, thus eliminating the possibility of flexibly changing the gas-mixture composition or increasing the concentration of a second gas; does not exclude the possibility of connecting an unspecified gas mixture including the mixtures not allowed for breathing; and does not provide for the use of an air mixture with oxygen or other gases. In the description of the apparatus, a particular design of the unit for setting the volume of respiratory mixture fed to the patient is not disclosed and the possibility of fully automatic control is not specified. HF ALVs, monitoring of ventilation indicators, introduction of a gas mixture directly to the trachea, and patient safety in case of failure of power supply and (or) compressed-air supply are not provided for. There is no possibility for actively mitigating the resistance to autonomous breathing.

## Summary of the invention

The object of the invention is to eliminate the mentioned disadvantages and to develop a new method for creating an inhalation effect and an apparatus for its implementation, which ensure an effective feeding of oxygen, atmospheric air or their mixtures, or two-component mixtures of oxygen with other gases, e.g. helium (He), xenon (Xe), and nitrogen oxide ($N_2O$) to a patient during inhalation treatment, inhalation or non-inhalation anesthesia, and intensive treatment via a rebreathing, including low-flow rebreathing, and non-rebreathing loop during artificial lung ventilation, including high-frequency (HF) lung ventilation, autonomous breathing, and their various combinations. The technical result consists in improving the efficiency and extending the functional capabilities of the inhalation effect.

In terms of the method, the set problem is solved and the technical result is achieved by that that according to the method of an inhalation effect on the organism, consisting in forming a respiratory mixture on the basis of oxygen and/or air and feeding same to a patient while ensuring the possibility of adding anesthetic vapors and/or aerosol of medicinal agents to the respiratory mixture, respiratory-mixture feeding mode is set with the help of the control module via a rebreathing or non-rebreathing loop through artificial lung ventilation and/or autonomous breathing, wherein flow and pressure of each gas is monitored by means of automatic adjustment and monitoring related to it by setting the isodromic flow of each gas in accordance with the signals from the control module, total flow of a respiratory mixture is independently measured and adjusted by setting the necessary flow as per signals of the control module, and also inhalation and exhalation lines are connected/disconnected to/from the atmosphere in line with the signals from the control module.

The respiratory mixture may be formed as a two-component mixture of oxygen with helium, xenon, or nitrogen oxide. In case of artificial lung ventilation, the respiratory mixture is preferably fed to the patient as jet high-frequency ventilation.

In terms of the device, the set problem is solved and the technical result is achieved by that that the apparatus for creating an inhalation effect on the human body, consisting of a respiratory-mixture formation device connectable to at least two gas sources, the primary unit with the unit for setting the volume of respiratory mixture fed to the patient, wherein the primary unit is connected to the primary outlet of the respiratory-mixture formation device via a filling line; inhalation and exhalation lines connected to the primary unit having diverters for connecting to the atmosphere, and which are integrated into a patient tee-fitting piece, control module, and means of automatic adjustment and monitoring; wherein the control unit consists of a display and a microprocessor controller interfacing with all of the means of automatic adjustment and monitoring, which, in turn, switch on the solenoid valve, flow meter, and gas-pressure-metering and gas-pressure stabilization facilities arranged in the respiratory-mixture formation device on each gas-source line, ensuring the possibility of independent determination of isodromic gas flow of each of the sources in line with the control-module signals; a controllable electric drive of the unit for setting the volume of a respiratory mixture fed to the patient, its proximity sensor, and an electromagnetic valve arranged in the primary unit, ensuring the possibility of setting the flow of the respiratory mixture fed to the patient in line with signals of the control unit; and also solenoid valves arranged on diverters for connecting to atmosphere of the inhalation and exhalation lines, wherein the valves allow for connection/disconnection of these diverters in line with the signals of the control unit.

At the inhalation line, a pneumatic anesthetic and/or medication sprayer may be installed. Respiratory-mixture formation device preferably contains an additional outlet for feeding a single-component gas, e.g., oxygen. The adjuster of the volume of breathing mixture supplied to the patient is preferably made as an elastic bellow, whose fixed wall tube is connected with the inhalation and filling lines, while the movable wall of the bellow is connected with the controllable electric drive and proximity sensor. The control module is preferably made with a possibility to form and maintain the rated pressure in a patient tee-fitting piece by setting the algorithm of creating an effect on the electric drive of the bellow. The apparatus may be provided with a high-frequency artificial-lung-ventilation module connectable to the respective independent outlet of a respiratory-mixture formation device and containing a stabilizer, a pressure gauge, a solenoid valve, and an injector located at the respiratory-tract entrance of a patient. Furthermore, a patient tee-fitting piece is connected to the suction tube of the injector and respiratory-mixture temperature and pressure sensors are installed at the injector outlet. Means for automatic adjustment, arranged at the exhaust line, preferably contain a non-return valve and a proportional solenoid valve, whose outlet is connected with the diverter for connecting to the atmosphere and with the filling line via the $CO_2$ absorber. A heater and a temperature sensor for the inhaled respiratory mixture are preferably installed in the inlet tube of a patient tee-fitting piece, and gas flow sensors and pressure sensors as well as the diverter of the respiratory-mixture sampling line are preferably arranged in the outlet tube. The heater preferably contains fast-response heating elements with adjustable heating intensity depending upon the gas flow. The filling line is preferably fitted with a non-return valve and the first safety valve and is connected with an elastic bag containing an inlet solenoid, a pressure sensor, and the second safety valve.

## Brief description of the drawings

Fig. 1 shows an internal pneumatic diagram of the respiratory-mixture formation device.

Fig. 2 shows a general pneumatic diagram of the device according to the invention.

### The best embodiment of the invention

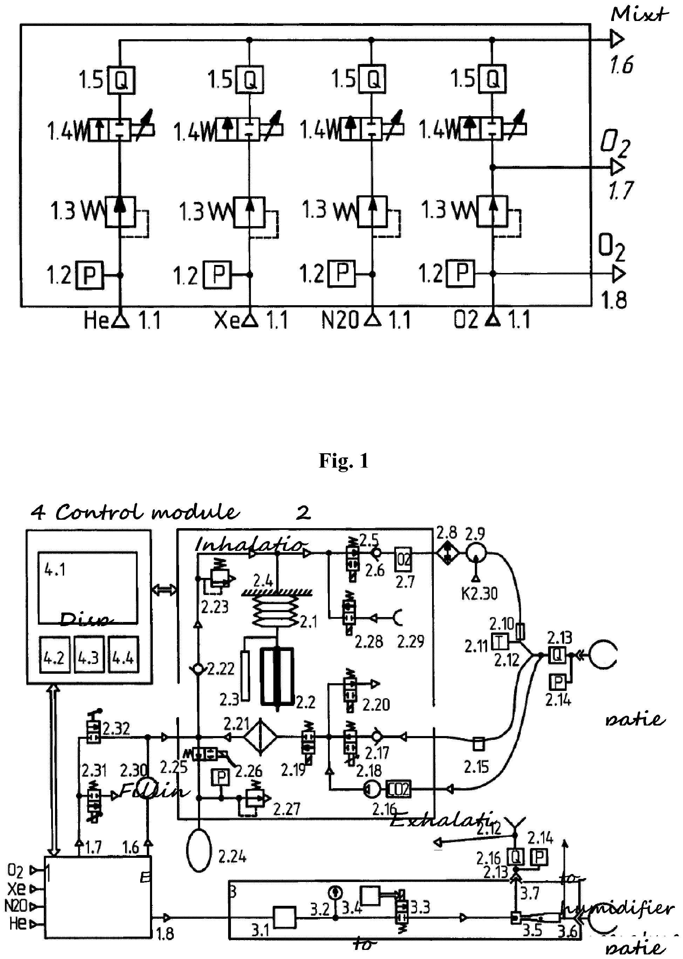

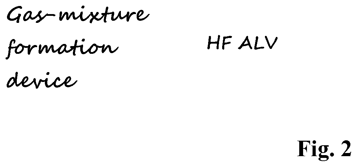

Unless otherwise is specifically stated in the description, special terminology corresponds to GOST 52423-2005, "Inhalation-anesthesia and artificial lung-ventilation devices. Terms and definitions." The common components shown in pneumatic diagrams in Fig. 1 and Fig. 2 comply with the requirements of the Unified system for design documentation 2.780-96, 2.781-96, 2.782, and 2.784-96, "Conventional graphical symbols". The applied gases are denoted by the generally accepted chemical symbols.

Within the present invention, the respiratory mixture should be understood as a gas or a gas mixture fed to patient for inhalation, wherein this mixture may be single-(pure oxygen or air), two-, and multicomponent. A non-rebreathing loop (NRL) contains a filling line, an inhalation and exhalation line up to the diverter for connecting to the atmosphere. A rebreathing loop (RL) consists of closed filling, inhalation, and exhalation lines in their entirety.

As seen in Fig.1, respiratory-mixture formation device (1) contains working lines according to the number of connectors (1.1) for connection of gas sources (as an example, Fig. 1 shows the diagram for formation device supplying $O_2$ and three other medical gases: He, Xe, and $N_2O$). Each line comprises: a supplied gas inlet pressure gauge (1.2), a pressure stabilizer (1.3), a normally closed (NC) proportional solenoid valve (SV) (1.4), and a supplied-gas flow meter (1.5). The outlets of all of the lines are connected with the common main outlet (1.6) of the respiratory mixture, and the pressure regulator outlet (1.3) in the $O_2$ line is connected with the additional $O_2$ stabilized pressure outlet (1.7). For feeding the high-frequency artificial lung-ventilation unit (HF ALV), the $O_2$ inlet to the respiratory-mixture formation device (1) is also directly connected with the respective independent outlet (1.8).

The primary unit (2) (Fig. 2) contains a unit for setting the volume of respiratory mixture, realized as a bellow (2.1) with a movable cover of which a stem of a controlled line electric drive (2.2) is connected, wherein the line electric drive contains the external or internal proximity sensor (2.3) for the movable bellow cover. The design advantage and preferability of such solution consists in that that the line electric drive (2.2) and sensor (2.3) ensure full correspondence of the character of contraction/expansion of the bellow (2.1) and, consequently, of the flow of the respiratory mixture passing through the general outlet (2.4) to the reciprocal movement of the line drive movement, which is set by the algorithm of action on the electric drive (2.2) calculated by the software that processes ventilation parameters set by the operator.

An inspiratory line is connected to the common outlet (2.4) of the bellow (2.1) containing a normally open (NO) SV (2.5), a non-return valve (2.6), and a concentration meter (2.7) for the $O_2$ in the inhaled mixture, which may contain an additional fast-response binary gas-mixture-composition sensor, based on the measurement of amplitude and frequency of resonant oscillations of the generator, whose piezoelectric transducer is placed in the tested gas environment.

The outlet of $O_2$ concentration meter (2.7) may connect the inhaled gas humidifier (2.8) and a pneumatic anesthetic and (or) medication sprayer (2.9). Directly upstream a patient tee-fitting piece (2.12), the heater (2.10) is installed, which, together with the temperature sensor (2.11), enabled the capability to maintain the predetermined high pressure of the inhaled mixture (e.g., $O_2$ + He).

The flow sensor (2.13) for a respiratory mixture is included in the common outlet of a patient tee-fitting piece (2.12); wherein a pressure sensor (2.14) of the respiratory mixture and the diversion of the diverter of the respiratory-mixture sampling line to a $CO_2$ meter (2.16) with the flow booster. The exhalation line is also connected with the tee-fitting piece (2.12), wherein the exhalation line includes a water-setting tank (2.15) and a fitted non-return valve (2.17). A fast-response proportional NO SV (2.18) is connected in series with the valve (2.17). Together with the NC SV (2.19) and NO SV (2.20) of the diverter for connecting an exhalation line to the atmosphere, it forms a switch for a breathing-loop type. As an embodiment of invention, SV (2.18), (2.19), and (2.20) may be replaced with one NO proportional SV with 3/2 switching and a nominal inside diameter of minimum 18-20 mm.

The outlet of NC SV (2.19) is connected, via the $CO_2$ absorber (2.21), with the filling line, which is fitted with the non-return valve (2.22) and the first safety valve (2.23) and is connected with the elastic bag (2.24). A NO SV (2.25) is installed at the inlet of the elastic bag (2.24); the bag pressure transducer (2.26) and the safety valve (2.27) are also located here. The inlet of SV (2.25) of the filling line is connected with the outlet (2.4) of the bellow (2.1). The NO SV (2.28) of the diverter for connecting an inhalation line to the atmosphere is also connected with the inlet (2.4), through which the atmospheric air may be fed to the bellow (2.1) or, through the filter (2.29), to the lungs of a patient in case of autonomous breathing. The bag (2.24) is connected with the main outlet (1.6) of the respiratory-mixture formation device (1) via the NC SV (2.25) and evaporator (2.30), if used, and also via the emergency $O_2$ supply button (2.32), directly with the additional oxygen outlet (1.7).

Anesthetic and/or medication sprayer (2.9) is also connected to the outlet (1.7) through the NC proportional SV (2.31).

The HF ALV module (3) is connected to the respective independent outlet (1.8) of the respiratory-mixture formation device (1) and contains a controllable $O_2$ pressure stabilizer (3.1), pressure gauge (3.2), NC SV (3.3), controlled electrical signal generator (3.4), and injector (3.5) located at the patient's respiratory tract entrance. When using a HF ALV, the sensor for temperature created by the moisturizer (2.8) and pressure sensor (2.14) are connected to the outlet (3.6) of the injector (3.5). A patient tee-fitting piece (2.12) with the gas-flow sensor (2.13) and the diverter of the sampling line of the respiratory mixture fed to the $CO_2$ sensor (2.16) are connected to the suction tube (3.7) of the injector (3.5).

The control module (4) consists of a display (4.1), which indicates the set, measured, and calculated values for ventilation and condition of a patient, functional curves, and requests for operator and also a microprocessor controller (4.2). The controller (4.2) interfaces with all of the means for automatic adjustment and monitoring, for which purpose it is electrically connected with all of the sensors, the electric drive (2.2), all of the SVs, measuring, alarming, humidifying, and heating devices, and also the display (4.1). The module (4) consists of a primary power-supply unit (4.3) and a backup power supply (4.4).

The proposed device operates as follows.

In every operation mode, compressed gases are fed to the respiratory-mixture formation device (1) via connectors (1.1), which are individual for each gas. In addition to its designated purpose, evaluation of the inlet pressure of each gas by the meters (1.2) allows for indicating on the display (4.1) which gases are connected at the given moment and preventing the feeding of other gases if $O_2$ is not supplied.

Pressure stabilizers (1.3) prevent the impact of inlet-pressure fluctuations of each gas as well as gas-flow variations in each channel upon the operation of the apparatus. This ensures preservation of calibration characteristics for all of the program-controlled proportional SVs (1.4), which prevent the added gases from penetrating into the device in case of intended or emergency switch-off of the apparatus.

Upon setting the necessary values for the respiratory mixture fed to the breathing loop and the $O_2$ concentration in it by the operator on the display 4.1 of the rebreathing loop (RL) or non-rebreathing loop (NRL), the program calculates and sets, via the respective proportional valves (1.4), the flow of $O_2$ and other predetermined gas using the algorithm:

$$Q_K = Q_{out}K_k/100$$

$$Q_g = Q_{out}(100 - K_k)/100,$$

where

$Q_k$ is the required $O_2$ flow;

$Q_g$ is the required flow of another gas, e.g., He;
$Q_{out}$ is the required flow of a gas mixture at the outlet of mixer (1.6);
$K_k$ is the percentage content of $O_2$ in the mixture at the common outlet (1.6).

The calculated values, $Q_k$ and $Q_g$ are indicated by the variable area flow meters shown on the display (4.1) and are controlled by the signals of flow meters (1.5). From the outlet (1.6), $O_2$ or its binary mixtures with other compressed gases connected to the device are fed to the primary unit (2). From the outlet (1.7), $O_2$ is fed to the breathing loop under stabilized pressure while bypassing the evaporator (2.30), through the NC emergency feed button (2.32) as well as through the proportional SV (2.31) to the sprayer (2.9). From the outlet (1.8), if necessary, $O_2$ is fed under inlet pressure to the HF ALV module (4)

If $O_2$ needs to be supplied as the mixture with the air during the NRL, $Q_{out}$ should be set less than the ventilation intensity (MV), the automatic pressure maintenance in the bag (2.24) is switched off, and in NRL, with the reduced pressure in the bag (2.24) to the admissible lower level, the necessary volume of environmental air is sucked in through the opened SV (2.28) and the filter (2.29) to the bellow (2.1) (in forced ventilation cycles) or to the patient's lungs (in autonomous breathing cycles), and the $O_2$ content in the inspired gas is monitored according to the indications of the sensor (2.7).

The operation of the primary unit (2) (Fig. 2) depends upon the selected operation mode.

1) In the controlled ALV mode with the predetermined volume, immediately after the device's switching on the position sensor (2.3) will ensure the automatic setting of the bellow (2.1) to the initial fully stretched position. Using the values of minute ventilation ($M_{uz}$), ventilation frequency (f), relative inhalation time ($T_i/T_c$) and relative time of the inhalation pause ($T_{pl}/T_i$) set by the operator on the display (4.1), the program calculates the bellow (2.1) compression parameters during the feeding of respiratory mixture according to the following algorithm:

$$T_{vd} = (T_i/T_c)-(60/f)(1-T_{pl}/T_i)$$

and

$$H = M_{uz}/[E_{ef}(1-T_{pl}/T_i)],$$

where

$T_{vd}$ is bellow (2.1) compression duration;
H is a bellow compression amplitude measured from the initial position;

$E_{ef}$ is the effective area of the bellow.

During the inhalation phase $T_i$ within the interval $T_{vd}$, a compressing bellow feeds the respiratory mixture to the lungs of the patient through the NO open SV (2.5), non-return valve (2.6), $O_2$ analyzer (2.7), humidifier (2.8), sprayer (2.9), and heater (2.10). Then, the inhaled gas is feed to the patient tee-fitting piece (2.12). Furthermore, the exhalation line is shut off with the NO closed proportional SV (2.18); the respiratory-mixture outlet to the atmosphere from the inhalation line is blocked by the NO closed SV (2.26), and the respiratory-mixture feeding from the bellow (2.1) to the bag (2.24) is blocked by the non-return valve (2.22).

If the limiting pressure in the breathing system ($P_{pred}$) is set above $P_{peak}$ (the maximum pressure in the breathing loop in the inhalation phase), then $P_{pred}$ is not reached and the apparatus feeds a predetermined respiratory volume to the patient. If the introduction of $U_g$ results in the condition of $P_{peak} = P_{pred}$, compression of the bellow (2.1) and gas feeding are stopped until the $T_i$ has been expired.

Upon completion of the inhalation phase, the SV (2.5) is closed and the exhalation phase starts. When setting the NRL, gas from the lungs is exhaled through the water collector (2.15), non-return valve (2.17), the fully NO opened proportional SV (2.18), and the NO opened SV (2.20) of the diverter for connecting to the atmosphere. In case of RL, the NO SV (2.20) remains closed and the exhaled gas is fed from the outlet of the proportional SV (2.18) through the NC opened SV (2.19) and the $CO_2$ absorber (2.21) through the opened SV (2.25) to the bag (2.24). If the PEEP is set, then when the reducing pressure reaches the preset PEEP according to the readings of the sensor (2.14), the fast-response proportional SV (2.18) will gradually close in order to maintain the indications of the sensor (2.14) at the predetermined PEEP level before the end of the exhalation phase $T_e$. Within the interval of $T_e$, the bellow (2.1) is stretched at a constant speed until reaching the initial position by taking the gas mixture from the bag (2.24) through the non-return valve (2.22) and NO SV (2.25).

At the end of $T_e$, the inhalation phase is repeated. During the breathing cycle, fresh gas mixture fed from the outlet (1.6) through the evaporator (2.30) (if used) fills the bag (2.24) but its direct feeding to the inhalation line is blocked by the non-return valve (2.22). Pressure in the bag (2.24) is maintained at its low level by the safety valve (2.27).

Supply of the predetermined gas volume is determined only by the amplitude and compression speed of the bellow (2.1), which is stabilized at a calculated level by the feedback comprising a sensor (2.3) (the effective area of the bellow is constant). Therefore, the value of inhaled volume $V_T$ is not affected neither by the composition of the gas mixture nor the pressure in the patient tee-fitting piece (2.12). Control is also simplified, owing to the fact that according to the generally known formula:

$$M_v - V_T f,$$

the ventilation frequency $f$ changed by the operator will not affect the ventilation intensity $M_V$ on account of the automatic reversely-proportional change of $V_T$. Since the change of the values $T_i/T_c$ and $T_{pl}/T_i$ by the operator will affect the feeding time $T_{vd}$, to maintain the predetermined values of $M_V$, $f$, and, respectively, $V_T$, the bellow compression-control algorithm will respectively change the supply compression speed by using the following algorithm:

$$Q_{iee} = Q_{iN}[C_N(1 - P_N)] / [C_N(1 - P_N)],$$

where

Q<sub>i</sub> is the fed respiratory-mixture flow;
$C = T_i/T_c$ is a relative inhalation time within the breathing cycle;
$P = T_{pl}/T_i$ is a relative time of the inhalation pause;
the index "$_{ee}$" is the changed value of the indicator;
the index "N" is the initial value of the indicator.

Connecting the device to the patient's breathing tract is often not completely leak-tight. Therefore, the actual exhaled volume $V_T$ is measured by integrating in time the sensor (2.13) signal, i.e., the respiratory-mixture flow in the exhalation phase through a patient tee-fitting (2.12). This allows for calculating the actual value of the ventilation intensity $M_u$ and indicating it and the exhaled volume $V_T$ on the display 4.1. Here, by the signals from the sensor (2.14), measured values of maximum, average, and minimum pressure of the breathing cycle as well as PEEP and the average pressure of the breathing cycle $P_{sr}$ are indicated too.

The use of NO SV (2.5), NO SV (2.18), NO SV (2.28), NO SV (2.20), NC SV (2.19), and non-return valves (2.6, 2.17) allows the patient to breathe autonomously through the inhalation and exhalation lines in ventilation modes that use the patient's autonomous breathing as well as in case of power supply failure in the apparatus.

2) To implement the mode of auxiliary controlled ALV, after setting the required values of $Q_i$, $T_i$, reference ventilation frequency $F_{an}$, sensitivity to the patient's respiratory effort $S_p$, and the PEEP on the display (4.1), the moment of end of the previous inhalation is determined based on the decrease of the flow of exhaled respiratory mixture $Q_e$ in the sensor (2.13) down to zero. The bellow (2.1) is set in the initial position, after which one of the algorithms for detecting the patient's inhalation attempt is activated.

In order to do this, the SV (2.5) and the proportional SV (2.18) are closed and the time of the breathing cycle $T_c = 60/F_{an}$ starts to be counted. If during this waiting time the patient's respiratory effort decreases the pressure in the patient tee-fitting (2.12) from zero or from the PEEP level to $S_p$, then SV (2.5) opens and the bellow (2.1) starts compressing by feeding the respiratory mixture to the patient's lungs at a predetermined flow $Q_i$. Feeding is continued until the $T_i$ expires or until the condition $P(t) = P_{pred}$ is met; then, SV (2.5) closes, proportional SV (2.18) fully opens, and the bellow (2.1) sets to its initial position once again.

When setting the RL, the exhaled gas mixture is supplied to the bag (2.24) through the open SV (2.19), absorber (2.21), and SV (2.25); when setting the NRL, the SV (2.19) is closed and the exhaled mixture goes out to the environment through the open SV (2.20) of the diverter for connecting to the atmosphere. Upon reaching the zero value of $Q_e$, the proportional SV (2.18) maintains the predetermined PEEP as described above for the controlled ALV and the respiratory-attempt detection algorithm is activated again. If it was not detected during $T_e$, then upon expiration of $T_c$, the next inhalation is made. Therefore, in this mode, the ventilation frequency is determined by the frequency of the patient's respiratory efforts; and in their absence or termination, by the predetermined Fan, while the minute ventilation is not stopped.

When using the NRL, the respiratory effort may also be detected by the predetermined gas flow appearing in the patient tee-fitting piece (2.12) and directed to the patient's lungs. In this case, simultaneously with starting counting the $T_c$, the SV (2.20) is closed and the open condition of SV (2.5), SV (2.18), and SV (2.19) is maintained. Through the evaporator (2.31) (if used) and the non-return valve (2.22), the inhalation and exhalation lines are washed with the respiratory mixture of the predetermined composition with the flow of

$$Q_{op} = p \cdot S_p,$$

where

Qop is a reference flow of the respiratory mixture ("FlowBy"), measured in 1/min;
p is a multiplier preset by the operator within the range, for example, from 2 to 5;
$S_p$ is sensitivity to the patient's inspiratory attempt, expressed in 1/min.

When the flow induced by the patient's inspiration reaches the predetermined level $S_p$, the line drive (2.2) starts compressing the bellow (2.1); the SV (2.5) remains open, proportional SV (2.18) closes, and the inhalation phase starts as described above.

The PEEP is reached in the same way as for the controlled ALV. If the patient's attempt is not detected, the inspiration phase will start upon the expiration of the predetermined $T_c$ in the same manner as for the detection of the pressure attempt. To simplify control, availability of the patient's inspiratory effort is demonstrated by the indication on the display (4.1) panel. The operator may also set the number of controlled ventilation cycles, after which the auxiliary controlled ALV mode is automatically switched on again.

3) To implement the autonomous breathing mode with the continuous positive pressure at the NRL, the line drive (2.2) holds the bellow (2.1) in a completely compressed state; the NO SV (2.5), NO SV (2.20), and the proportional NO SV (2.18) are constantly open. The inhalation and exhalation lines' washing with the respiratory mixture of a predetermined composition through the formation device (1) with the initial flow is automatically switched on, and the NO proportional SV (2.18) is dynamically set to a position ensuring the predetermined CPAP ventilation level at the outlet of the patient tee-fitting piece (2.12). Then, the respiratory-mixture flow will be automatically adjusted so that to make the pressure fluctuations in the tee-fitting piece (2.12) as a result of patient's autonomous breathing reflected by the sensor (2.14) symmetrical with respect to the predetermined constant CPAP ventilation level.

To implement the alternative CPAP ventilation method during the RL, the SV (2.5) and SV (2.19) remain open; SV (2.20) and safety valve (2.27) are closed; SV (2.19) and SV (2.25) are opened. The line drive (2.2) completely compresses the bellow (2.1) and then sets it to a partially stretched position. Through the feeding of breathing mixture, the breathing loop becomes filled, including the bellow (2.1) and the bag (2.24), until the predetermined CPAP ventilation level is set within them; and then its level is stabilized automatically. Pressure reduction in the breathing loop during the patient's inhalation is blocked by the compression of the bellow (2.1) by the line drive (2.2). Similarly, the bellow (2.1) stretching blocks the pressure increase in the system as a result of the patient's exhalation. Therefore, the pressure in the tee-fitting piece (2.12) is supported at the predetermined constant CPAP ventilation level and the reduction of this level as a result of $O_2$ absorption by the patient, $CO_2$ absorption in the absorber (2.21), and possible leak through the breathing system is compensated by replenishing of the breathing loop with a fresh mixture from the respiratory-mixture formation device (1). Furthermore, the inhalation and exhalation resistance of the apparatus is automatically reduced practically to zero, i.e., the breathing is only determined by the resistance of the patient's respiratory tract.

4) To implement the jet HF ALV from the outlet (1.8), compressed $O_2$ is supplied to the pressure regulator (3.1) set by the operator. Oxygen is supplied to the NC SV (3.3) inlet of 2/2 type at a stabilized pressure measured by the pressure gauge (3.2). The electric control signals of the given SV are generated in the formation device (3.4) allowing for independently setting f and $T_i/T_c$. Through a flexible tube, pneumatic pulses are supplied to the injector (3.5) nozzle. Its suction tube (3.7) is connected with the common outlet of tee-fitting piece (2.12). SV (2.5), (2.26), (2.18), and (2.20) are maintained open in this mode; and the bellow (2.1) remains still. Therefore, $O_2$ supplied in the inhalation phase to the patient's lungs through the injector (3.5) nozzle is complemented with air passed through the filter (2.29), SV (2.28, 2.5), valve (2.6), and humidifier (2.8). The signal of the temperature sensor fitted at the outlet (3.6) of the injector is sent to the humidifier (2.8), which humidifies and heats the inhaled respiratory mixture. Therefore, the effective HF ALV is ensured by joint application of the HF ALV module (3) and other modules of the apparatus.

The apparatus control in the HF ALV mode is substantially simplified by the pressure curve indicated on the display (4.1) and by measurement with the flow sensors (2.13) and pressure sensors (2.14) of the ventilation indicators, including the inhaled volume and minute ventilation.

The described technical solutions ensure the following important safety measures of the device:

- the possibility of the patient's autonomous breathing environmental air in case of failure of the device and (or) termination of gas-mixture supply from the gas-mixture formation device (1), ensured by the respective selection of the type (NO or NC) of all of the SVs and proper distribution of the gas flows within the breathing loop with the help of non-return valves (2.6, 2.17, 2.22); measurement and displaying on the display (4.1) of the ventilation indicators including the composition of $CO_2$ in the exhaled gas as well as the concentration of $O_2$ in the inhaled gas mixture;
- sound and visual alarm in case the measured parameters go over the safety threshold set by the operator on the display (4.1),
- restriction of the maximum pressure in the breathing loop with a safety valve (2.23) with activation of alarm and immediate termination of gas supply to the patient's lungs if $P_{peak}$ reaches the maximum allowable value set by the operator;
- the automatic output on the display (4.1) of warning and explanatory messages;
- continuous operation of the device from a backup power-supply source as a result of energy-saving mode of the bellow drive.

In view of the above-mentioned, the suggested method and device for its implementation ensure the use of new treatment methods by feeding to the patient via inhaling of gas mixtures of different composition, including oxygen mixtures with different gases, e.g., helium, xenon, or nitrogen oxide, with simultaneous heating of the respiratory mixture to at least +70 °C, and injection of aerosols of anesthetic and/or medicinal agents in different applications, such as in inhalation treatment, in inhalation and non-inhalation anesthesia, and in intensive treatment, and resuscitation, in cases of patient's autonomous breathing, ALV, and in different combinations thereof as well as with the use of HF ALV. In all cases, the absence of influence on the ventilation indicators of the respiratory mixture composition and change of the patient's lung characteristics as well as the monitoring of ventilation indicators and patient condition are ensured. High safety level for the patient safety and the ease of control are achieved along with the possibility to extend functional capabilities while preserving its structure and the integrated design solutions allow supplying more specific devices at the request of the customer.

### *Industrial applicability*

The invention allows for considerably improving the efficiency of inhalation treatment of various diseases and conditions in pulmonology, therapy, intensive treatment, anesthesiology, and sports medicine and is provided with new integral technical solutions while employing the opportunities of microelectronic technology and modern components. A modular structure of the apparatus simplifies its manufacture, ensures the ease of setting it up, and operation in various operating modes. When using the invention, monitoring of the ventilation indicators and patient condition with achieving his high safety level may be ensured; in sports medicine a problem of rehabilitation and training of athletes with the background of extreme loads and increased ventilation indicators is solved.

### Claims

1. A method of the inhalation effect on the human body consisting in forming a respiratory mixture on the basis of oxygen and/or air and feeding same to a patient with the addition of anesthetic vapors and/or of an aerosol of medicinal agents to the respiratory mixture, **characterized by** that the respiratory-mixture feeding mode is set with the help of the control module via the rebreathing or non-rebreathing loop through artificial lung ventilation and/or autonomous breathing; wherein the flow and pressure of each gas is monitored by means for automating adjustment and monitoring connected with it by setting the isodromic flow of each gas in accordance with the signals from the control module; wherein the total flow is independently measured and controlled by setting the necessary flow as per signals of the control module; and also inhalation and exhalation lines are connected/disconnected to/from the atmosphere in line with the signals from the control module.

2. A method according to claim 1, wherein the respiratory mixture is generated as a two-component mixture of oxygen with helium, xenon, or nitrogen oxide.

3. A method according to claim 1, wherein in case of artificial lung ventilation, the respiratory mixture is fed to the patient as jet high-frequency ventilation.

4. A device for inhalation effect on the human body, consisting of a respiratory-mixture formation device, which is connectable to at least two gas sources; the primary unit with the unit for setting the volume of respiratory mixture fed to the patient, wherein the primary unit is connected to the main outlet of the respiratory-mixture formation device via a filling line; inhalation and exhalation lines connected to the primary unit, wherein the lines are fitted with diverters for connecting to the atmosphere and integrated into a patient tee-fitting piece; a control module; and means for automatic adjustment and monitoring; **characterized by** that the control module consists of a display and a microprocessor controller, which interfaces with all of the means for automatic adjustment, which, in turn, switch on the solenoid valve, flow meter, and gas-pressure metering and stabilization facilities arranged in the respiratory-mixture formation device on each of the gas sources line, ensuring the possibility of independent determination of isodromic gas flow from each of the sources in line with the control-module signals; a controllable electric drive of the unit for setting the volume of respiratory mixture fed to the patient, its position sensor, and electromagnetic valve arranged in the primary unit, ensuring the possibility of setting the flow of respiratory mixture fed to the patient in line with signals of the control module; and also solenoid valves arranged on diverters for connecting to the atmosphere of the inhalation and exhalation lines allowing to connect/disconnect these diverters in line with the signals of the control module.

5. A device according to claim 4, **characterized by** that a pneumatic anesthetic and/or medication sprayer is installed at the inhalation line.

6. A device according to claim 4, **characterized by** that a respiratory-mixture formation device includes an additional outlet for feeding single-component gas, e.g., oxygen.

7. A device according to claim 4, **characterized by** that the unit for setting the volume of respiratory mixture fed to the patient is made in the form of an elastic bellow, whose fixed wall tube is connected with the inhalation and filling lines; wherein the movable wall of the bellow is connected with the controllable electric drive and proximity sensor.

8. A device according to claim 7, **characterized by** that the control module has a possibility to generate and maintain the rated pressure in a patient tee-fitting piece by setting the algorithm of influencing the electric drive of the bellow.

9. A device according to claim 4, **characterized in that** it is has a high-frequency artificial lung ventilation unit, which is connectable to the respective independent outlet of a respiratory-mixture formation device and which contains a stabilizer, a pressure gauge, a solenoid valve, and an injector located at the entrance of the patient's respiratory tract.

10. A device according to claim 9, **characterized by** that a patient tee-fitting piece is connected to the suction tube of the injector and respiratory-mixture temperature and pressure sensors are installed at the injector outlet.

11. A device according to claim 4, **characterized by** that means for automatic adjustment and monitoring, arranged at the exhalation line, consist of a non-return valve and a solenoid proportional valve, whose outlet is connected with a diverter for connecting to the atmosphere and, via the $CO_2$ absorber, with the filling line.

12. A device according to claim 4, **characterized by** that a heater and a temperature sensor for the inhaled respiratory mixture are fitted in the inlet tube of a patient tee-fitting piece and flow sensors and pressure sensors as well as the diverter of the respiratory-mixture sampling line are preferably arranged in the outlet tube.

13. A device according to claim 12, **characterized by** that a heater has fast-response heating elements with controllable heating intensity depending upon the gas flow.

14. A device according to claim 4, **characterized by** that the filling line is preferably filled with a non-return valve and the first safety valve and is connected with an elastic bag containing an inlet solenoid, a pressure sensor, and the second safety valve.

Fig. 1

Gas-mixture
formation          HF ALV
device

**Fig. 2**

## INTERNATIONAL SEARCH REPORT

| | | International application No. |
|---|---|---|
| | | PCT/RU 2017/000390 |

**A. CLASSIFICATION OF SUBJECT MATTER**
A61M 16/12 (2006.01); A61M 16/01 (2006.01)

According to International Patent Classification (IPC) or to both national classification and IPC

**B. FIELDS SEARCHED**

Minimum documentation searched (classification system followed by classification symbols)
A61M 16/00-16/12, 15/00

Documentation searched other than minimum documentation to the extent that such documents are included in the fields searched

Electronic data base consulted during the international search (name of data base and, where practicable, search terms used)
USPTO, DEPATISnet, Esp@cenet, Information Retrieval System of FIPS, EAPATIS, K-PION, SIPO, PubMed, SCOPUS

**C. DOCUMENTS CONSIDERED TO BE RELEVANT**

| Category* | Citation of document, with indication, where appropriate, of the relevant passages | Relevant to claim No. |
|---|---|---|
| D, Y | RU 2072241 C1 (PANINA ELENA VLADIMIROVNA) 27.01.1997, fig. 1, the abstract, the claims | 1-14 |
| Y | US 2002/0023643 A1 (KARSTEN HOFFMANN) 28.02.2002, fig. 1, par. [0003], [0017] - [0028] | 1-14 |
| Y | RU 2207886 C2 (ASTRAZENEKA AB et al.) 10.07.2003, fig. 1, the abstract | 4-14 |
| Y | RU 2001110752 A (PANINI ALEKSANDR ANDREEVICH et al.) 10.03.2003, the claims, the abstract | 2 |
| Y | RU 2162682 C1 (KOZLOV ALEKSANDR JUREVICH) 10.02.2001, the claims | 3, 9-10 |

☐ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31 August 2017 (31.08.2017) | 14 September 2017 (14.09.2017) |

| Name and mailing address of the ISA/ RU | Authorized officer |
|---|---|
| Facsimile No. | Telephone No. |

Form PCT/ISA/210 (second sheet) (April 2005)

**EP 3 479 862 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2072241
- RU 2291718
- US 6553990 B
- RU 2146536